# DEMANDE DE BREVET EUROPEEN

(11) **EP 2 277 476 A1**
(43) Date de publication de la demande: **26.01.2011**
(21) Numéro de dépôt: 10170651.3
(22) Date de dépôt: 23.07.2010
(51) Int. Cl.: A61F 2/42

(54) **Dispositif implantable d'interposition pour la refection d'une articulation phalangienne ou metacarpophalangienne et ensemble chirurgical comprenant un tel dispositif**

(30) Priorité: 23.07.2009 FR 0955156
(71) Demandeur: Textile Hi-Tec (T.H.T.), 34220 Verreries de Moussans (FR)
(72) Inventeur: Houard, William, 81270, LABASTIDE ROUAIROUX (FR)
(74) Mandataire: Cochonneau, Olivier

(57) **Abrégé**

Le dispositif implantable concerne la réfection d'une articulation phalangienne ou métacarpo-phalangienne présentant une déficience en cartilage. Il comporte une broche de fixation, pour sa fixation dans l'un des os de l`articulation, surmontée d'une base d'appui apte à remplacer le cartilage déficient.

La broche de fixation (2) est formée d'une tête (4) et d'une tige (5). Au moins la tête (4) et la base d'appui (3) sont dans des matériaux différents, celui de la base d'appui (3) étant plus déformable sous contrainte que celui de la tête (4).

La base d'appui (3) présente une ouverture centrale dont la configuration et celle de la tête (4) sont déterminées en sorte que la base d'appui (3), lors de son emboîtement à force sur la tête, se déforme pour adopter une courbure concave de rayon R1.

## Description

La présente invention concerne le domaine des implants ou prothèses articulaires qui sont dits d'interposition en ce qu'ils comportent un élément destiné à être implanté à l'interface articulaire entre deux os.

La mise en oeuvre d'un implant articulaire d'interposition intervient dans les indications arthrosiques lorsqu'une base osseuse de l'articulation présente une déficience en cartilage due notamment à la dégradation de celui-ci. Outre l'amélioration de la mobilité de l'articulation, la mise en place d'un implant articulaire d'interposition vise également à limiter partiellement ou totalement la douleur générée par l'arthrose.

On a déjà proposé des prothèses pour articulation métacarpo phalangienne et inter-phalangienne de la main ou du pied qui présente la particularité de comporter deux têtes de fixation reliées par une pièce de liaison faisant office d'articulation, chaque tête de fixation étant destinée à être implantée dans l'un des deux os adjacents de l'articulation à restaurer. La pièce de liaison doit présenter une mobilité similaire à celle recherchée pour l'articulation. De telles prothèses sont connues notamment par les documents W0.96/25129, W0.2005/067822 et W0.2007/114.769.

On connaît également des prothèses, parfois dénommées prothèses boutons, qui ne comportent qu'une seule broche de fixation associée à une cupule ou bouton, ladite broche de fixation étant destinée à fixer ladite cupule à l'extrémité d'un des deux os de l'articulation, tandis que la cupule vient en contact avec la surface articulaire de l'os adjacent. Pour sa fixation par brochage axial, la cupule est percée d'un trou central permettant le passage de la broche de fixation.

Il est à noter que le document EP.1.637.095 cherche à éviter la fixation par brochage axial et donc à éliminer purement et simplement la broche de fixation, l'immobilisation de la cupule étant obtenue grâce à des moyens d'immobilisation qui viennent en appui contre au moins l'une des surfaces articulaires ; plus précisément, les moyens d'immobilisation sont conformés pour empêcher la rotation de la cupule sur elle-même par rapport à son axe longitudinal. Par exemple, s'agissant de moyens d'immobilisation intégrés à la cupule elle-même, ladite cupule comporte une pluralité de faces planes, sensiblement inclinées les unes par rapport aux autres, de manière à former un polyèdre, avec éventuellement un bourrelet de raccordement entre deux faces planes adjacentes. Un tel implant articulaire d'interposition est particulièrement complexe à fabriquer pour obtenir d'une part la mobilité de l'articulation et l'immobilisation recherchée.

L'implant de la présente invention reste dans le domaine connu des implants composés d'une cupule et d'une broche de fixation, comme indiqué précédemment. Le but visé est de faciliter d'une part la fabrication dudit implant et d'autre part sa mise en place par le praticien tout en apportant la mobilité recherchée pour l'articulation et une parfaite adaptation à la surface articulaire contre laquelle la cupule vient s'appliquer.

L'ensemble de ces objectifs est parfaitement atteint par le dispositif implantable pour la réfection d'une articulation phalangienne ou métacarpo-phalangienne qui présente une déficience en cartilage, ledit dispositif comportant de manière connue une broche de fixation, pour sa fixation dans l'un des os de l'articulation, surmontée d'une base d'appui apte à remplacer le cartilage déficient.

De manière caractéristique, selon la présente invention :
- la broche de fixation étant formée d'une tête et d'une tige, au moins la tête de la broche et la base d'appui sont dans des matériaux différents, celui de la base d'appui étant plus déformable sous contrainte que celui de la tête de la broche de fixation ;
- la base d'appui présente une ouverture centrale pour l'insertion, par emboîtement à force, et le blocage de la tête de fixation ;
- les configurations de l'ouverture centrale de la base d'appui et de la tête de la broche de fixation sont déterminées en sorte que la base d'appui, lors de son emboîtement à force sur ladite tête, se déforme pour adopter une courbure concave de rayon R1.

Cette courbure concave doit permettre la congruence avec la surface articulaire contre laquelle est appliquée la base d'appui, congruence qui est encore améliorée du fait de la capacité de déformation de la base d'appui, rendant celle-ci susceptible d'épouser les irrégularités de la surface articulaire contre laquelle elle est appliquée.

De préférence, la tête de la broche de fixation a sa face supérieure qui présente une courbure concave de rayon R2, sensiblement égale à R1. Lorsque la broche de fixation et la base d'appui sont assemblées, la face extérieure de la base d'appui est dans le prolongement de la face supérieure de la tête de la broche de fixation. Ainsi il n'y a pas de discontinuité dans le contact entre la surface articulaire et l'implant, ce contact ayant lieu à la fois sur la base d'appui et la face supérieure de la tête de la broche de fixation, toutes deux de courbure concave.

Le document EP.1.637.095 mentionne que les implants articulaires d'interposition formés d'une cupule et d'une broche de fixation sont des implants à fixation temporaire qui doivent être retirés une fois que les tissus fibreux sont reconstitués. Ceci nécessite une nouvelle intervention chirurgicale.

Avantageusement, selon la présente invention, la base d'appui et la broche de fixation sont toutes deux dans des matériaux résorbables. Il s'agit en particulier d'un copolymère d'acide polylactique (PLA) et de caprolactone pour la base d'appui et d'acide polylactique lévogyre et dextrogyre (PLDL) pour la broche de fixation. Ainsi, le matériau résorbable de la base d'appui doit avoir une durée de résorption suffisante pour que les tissus fibreux soient totalement reconstitués. Il doit aussi avoir une déformation sous contrainte plus importante que le matériau résorbable de la broche de fixation, pour permettre la déformation de la base d'appui lors de l'emboîtement à force de la tête de la broche de fixation dans ladite base d'appui.

Dans une variante de réalisation permettant cette déformation, la tête de la broche de fixation est reliée à la tige de fixation par un épaulement annulaire, formant butée pour la face inférieure de la base d'appui, lors de l'assemblage. De plus ladite tête a, en section transversale, des dimensions externes qui sont, dans la zone proche dudit épaulement, supérieures à celles internes de l'ouverture centrale et qui sont, dans la zone opposée audit épaulement, inférieures à celles internes de l'ouverture centrale. Ainsi la tête de la broche de fixation, à proximité de l'épaulement, comporte de la matière excédentaire, comparativement à la zone de l'ouverture centrale dans laquelle elle va être disposée lors de l'assemblage. Etant donné que la matière dont est constituée la base d'appui est plus déformable sous contrainte que celle dont est constituée la tête de la broche de fixation, cet excédent de matière de la tête de la broche de fixation va avoir tendance à repousser la matière de la base d'appui lors de sa pénétration, générant des contraintes importantes ayant pour effet de provoquer une déformation de la base d'appui, déformation qui est rendue possible grâce au fait que, dans la zone opposée à l'épaulement, la tête de la broche de fixation comporte moins de matière que l'ouverture centrale. Cette déformation se traduit par une mise en concavité de la base d'appui.

Dans une variante de réalisation, la tête de la broche de fixation comporte, à proximité immédiate de l'épaulement annulaire, une première portion cylindrique de hauteur H1, prolongée par une seconde portion tronconique divergente de hauteur H2 et d'angle au somme A2 et l'ouverture centrale de la base d'appui comporte une première portion cylindrique de hauteur H'1 supérieure à H1 et une seconde portion tronconique de hauteur H'2 et d'angle au somme A'2 supérieur à A'1.

Dans un exemple préféré de réalisation, H'1 = 2 x H1, A'2 = 1,5 A2 et H1 + H2 est sensiblement égal à H'1 + H'2. Par exemple H1 = 0,2 mm, H'1 = 0,4 mm, H1 + H2 = 2,45 mm, H'1 + H'2 = 2,5 mm, A2 = 60° et A'2 =90°.

Dans une variante de réalisation, la base d'appui est une simple rondelle annulaire, dont les deux faces supérieure et inférieure sont parallèles, avant l'assemblage avec la broche de fixation, le bord périphérique de ladite rondelle étant de préférence arrondi. Elle est donc de fabrication particulièrement simplifiée.

Pour faciliter son ancrage dans l'os, la tige de la broche de fixation comporte des crans. De préférence, le cran le plus proche de la tête de la broche fait office de butée pour la face inférieure de la base d'appui lors de l'assemblage des deux pièces.

Dans une variante de réalisation, chaque cran présente la forme d'un tronc de cône, convergent vers l'extrémité de la tige, dont la petite base a un diamètre D2 qui est égal ou inférieur au plus petit diamètre D'1 de l'ouverture centrale de la base d'appui et dont la grande base a un diamètre D3 qui est supérieur au diamètre D'1.

Dans un exemple de réalisation D2 et D'1 sont de l'ordre de 2,7 mm et D3 de l'ordre de 3,5 mm. La pénétration de la tige de la broche de fixation dans l'ouverture centrale de la base d'appui ne peut se faire qu'à force et grâce à la déformabilité sous contrainte du matériau constitutif de la base d'appui.

C'est un autre objet de l'invention que de proposer un ensemble chirurgical incorporant au moins un dispositif implantable pour la réfection d'une articulation phalangienne ou métacarpo-phalangienne, comportant les caractéristiques mentionnées ci-dessus et qui est destiné à faciliter le travail du praticien.

Cet ensemble comporte d'une part un dispositif précité dans lequel la base d'appui est une rondelle annulaire de diamètre D0 et d'autre part un instrument de découpe apte à réaliser la découpe de ladite rondelle selon un diamètre D1 inférieur à D0. Bien sûr le diamètre D1 dont il s'agit est celui qui est déterminé par le praticien au regard des paramètres propres de son patient.

De préférence, cet ensemble comporte également un jeu de gabarits destiné à permettre au praticien de mesurer, directement sur son patient, le diamètre D1 en question.

Dans un mode préféré de réalisation, l'instrument de découpe est une pince emporte-pièce dont un premier mors est muni d'une platine circulaire équipée d'un téton de centrage pour le placement de la base d'appui et dont le second mors est muni d'un outil de coupe circulaire ; de plus les deux mors ont un déplacement parallèle.

La présente invention sera mieux comprise à la lecture de la description qui va être faite d'un exemple préféré de réalisation d'un dispositif résorbable d'interposition phalangienne et métacarpophalangienne, illustré par le dessin annexé dans lequel :
La figure 1 est une vue schématique en perspective du dispositif, la base d'appui étant assemblée à la broche de fixation ;
La figure 2 est une représentation illustrant partiellement le squelette d'une main dans laquelle sont implantés deux dispositifs ;
La figure 3 est une représentation en coupe transversale de la broche de fixation et
La figure 4 est une représentation en coupe transversale de la base d'appui avant son assemblage avec la broche de fixation.
La figure 5 est une représentation schématique partielle en coupe d'une pince de découpe pour la base d'appui et
La figure 6 est une représentation en vue de dessus d'un exemple de gabarit pour la mesure du diamètre D1 de la base d'appui.

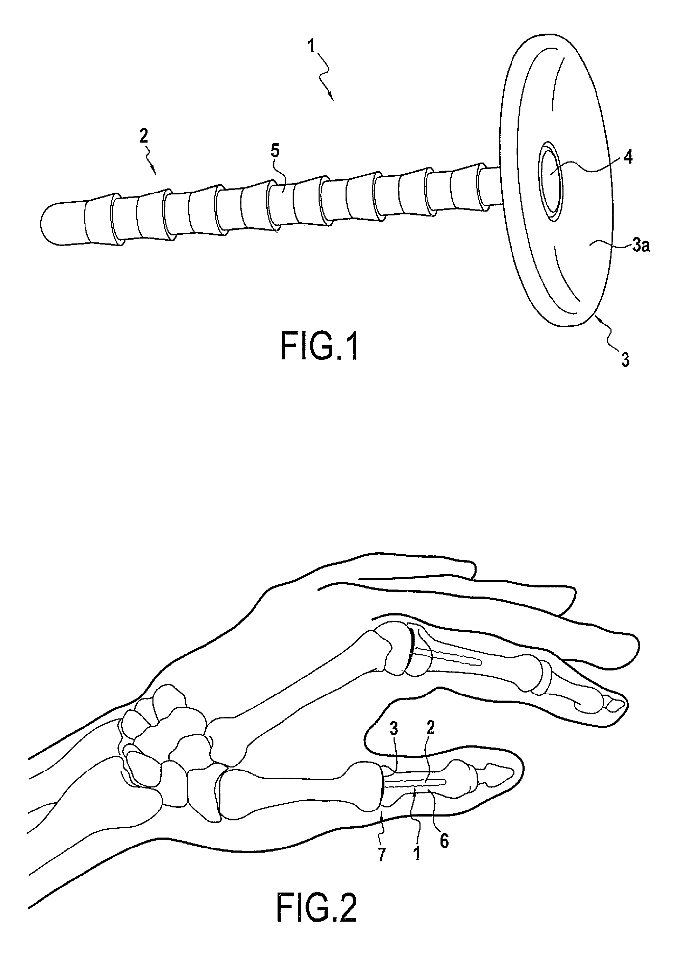

Le dispositif implantable 1 de la présente invention est utilisé pour la réfection d'une articulation phalangienne ou métacarpophalangienne qui présente une déficience en cartilage. Ce dispositif 1 comporte deux pièces distinctes à savoir une broche de fixation 2 et une base d'appui 3. La broche de fixation 2 comporte une tête 4 et une tige 5, cette dernière étant destinée à pénétrer dans l'un des os 6 de l'articulation 7 à restaurer, tandis que la base d'appui 3 est destinée à remplacer au moins partiellement le cartilage déficient de ladite articulation 7. Sur la figure 1, le dispositif 1 est montré dans la configuration qu'il présente lorsqu'il est implanté, la base d'appui 3 présentant une face supérieure 3a de courbure concave.

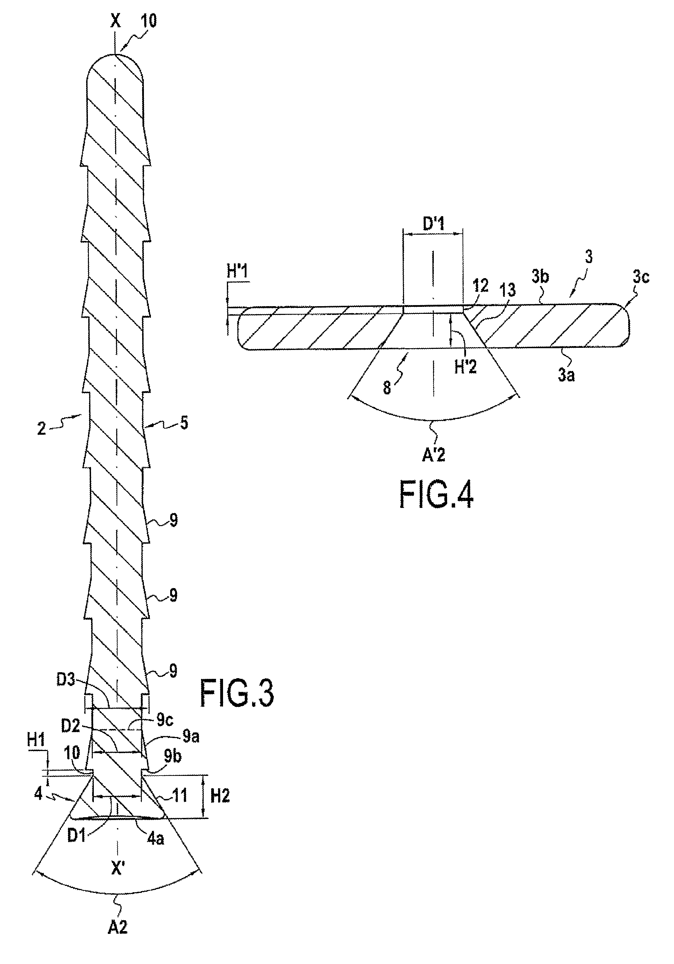

Sur la figure 4, la base d'appui est représentée dans la configuration qu'elle présente avant son assemblage avec la broche de fixation 2. Il s'agit d'une simple rondelle annulaire dont la face supérieure 3a est parfaitement plane et parallèle à la face inférieure 3b et dont le bord extérieur 3c est arrondi.

La mise en concavité de la base d'appui 3 se fait lors de l'assemblage à force de la base d'appui 3 à la tête 4 de la broche de fixation 2. Pour cela, d'une part au moins la tête 4 de la broche 2 est dans un matériau qui est moins déformable sous contrainte que celui de la base d'appui 3 et d'autre part les dimensions externes de la tête 4 de la broche 2 de fixation diffèrent des dimensions internes de l'ouverture centrale 8 de la base d'appui 3 en sorte que la pénétration à force de ladite tête 4 dans ladite ouverture centrale 8 génère des contraintes telles que la base d'appui 3 se déforme lors de l'assemblage de la broche 2 et de la base d'appui 3 pour adopter une courbure concave de rayon R1.

La valeur souhaitée pour ce rayon de courbure R1 peut varier en fonction des os concernés et le type de pathologie. Généralement R1 est de l'ordre de 20 mm.

De préférence la face supérieure 4a de la tête 4 de la broche de fixation 2 présente elle-même une courbure concave de rayon R2, sensiblement égal au rayon de courbure R1. Ainsi lorsque la broche de fixation 2 et la base d'appui 3 sont assemblées, la face supérieure 3a de la base d'appui 3 est dans le prolongement de la face supérieure 4a de la tête 4 de la broche de fixation. On a donc une surface de contact qui est continue entre le dispositif 1 implantable et la surface articulaire, ce qui permet une congruence parfaite entre ledit dispositif et ladite surface articulaire, congruence qui est encore améliorée du fait de la capacité de déformation de la base d'appui 3, rendant celle-ci susceptible d'épouser les irrégularités de la surface articulaire contre laquelle elle est appliquée. Dans l'exemple qui est illustré aux figures 3 et 4, la tige 5 de fixation présente une pluralité de crans 9 qui sont tous de forme tronconique convergeant vers l'extrémité 10 de la tige 2, qui est à l'opposé de la tête 4.

La grande base 9b du cran 9a de diamètre D3, qui est immédiatement adjacente à la tête 4, délimite un épaulement annulaire qui, lors de l'assemblage, fait office de butée pour la face inférieure 3b de la base d'appui 3. La petite base 9a du cran 9 a un diamètre D2 qui est égal au diamètre D4 de la tige 2.

La tête 4 de la broche 2 de fixation comporte, à partir de la grande base 9b du dernier cran 9a une première portion cylindrique 10 d'une hauteur H1, ladite hauteur étant prise selon l'axe longitudinal XX' de la broche 2, le diamètre D1 de cette première portion cylindrique est égal ou sensiblement égal au diamètre D2 de la petite base 9c du cran 9a. Cette première portion cylindrique 10 est prolongée par une seconde section tronconique 11 d'une hauteur H2 et d'angle au sommet A2. L'ouverture centrale 8 de la base d'appui 3 comporte, depuis sa face inférieure 3b jusqu'à sa face supérieure 3a, une première portion cylindrique 12, de diamètre D'1 et de hauteur H'1 supérieure à H1, prolongée par une seconde portion tronconique 13 divergente, de hauteur H'2 et d'angle au somme A'2 supérieur à A2. Les deux portions cylindriques 10 et 12 ont sensiblement le même diamètre.

Dans un exemple précis de réalisation, donné à titre non exhaustif, D1, D'1, D2 et D4 sont de 2,7 mm, D3 est de 3,5 mm, H1 de 0,2 mm, H'1 de 0,4 mm, A2 de l'ordre de 60° et A'2 de l'ordre de 90°. La hauteur totale (H1 + H2) de la tête 4 est de 2,45 mm et celle (H'1 + H'2) de la base d'appui est de 2,5 mm.

La formation du dispositif 1, par assemblage de la base d'appui 3 et de la broche de fixation 2, se fait en introduisant l'extrémité 10 de la broche de fixation 2 dans l'ouverture centrale 8 depuis la face supérieure 3a de la base d'appui 3 jusqu'à ce que la tête 4 de la broche 2 vienne en butée contre l'épaulement formé par la grande base 9b du premier cran 9a. Le passage de chaque cran au niveau de la première portion cylindrique 12 de la base d'appui 3 ne peut se faire qu'à force, ce qui est rendu possible grâce à la déformabilité du matériau constitutif de ladite base d'appui 3. De plus l'excédent de matière, dû au fait que la hauteur H1 de la première portion cylindrique 10 de la tête 4 est inférieure à la hauteur H'1 de la première portion cylindrique 12 de la base d'appui, provoque lors du calage en butée de ladite base d'appui 3 une déformation de celle-ci, provoquant une courbure concave dont le rayon R1 est sensiblement égal à celui R2 de la face supérieure de la tête 4.

On comprend qu'il est possible de faire varier la valeur du rayon de courbure R1 en choisissant de manière adéquate l'excédent de matière de la tête 4 à proximité de l'épaulement 9b et donc les différences de dimensionnement entre la tête 4 et l'ouverture centrale 8 de la base d'appui 3. On comprend également que cette mise en concavité de la base d'appui 3 n'est possible qu'en ménageant également une zone, à proximité de la face supérieure 3a de la base d'appui 3, dans laquelle la tête 4 est en déficit de matière par rapport à la zone correspondante de l'ouverture centrale 8 de la base d'appui de manière à compenser corrélativement la déformation concave obtenue. Cette déformation concave entraîne corrélativement une diminution de l'angle au sommet A'2 de la base d'appui jusqu'à l'obtention d'un angle qui soit sensiblement égal à A2, l'angle au sommet de la tête 4 de la broche de fixation 2.

Dans un mode préféré de réalisation, le dispositif implantable d'interposition 1 est résorbable de manière à éviter d'avoir à être retiré ultérieurement, la durée de résorption devant être suffisante pour que les tissus fibreux soient totalement reconstitués.

Il convient de sélectionner, parmi tous les matériaux résorbables, un matériau pour la base d'appui qui ait une certaine capacité de déformation sous contrainte, contrairement à ce qui est recherché pour la broche de fixation elle-même ou tout au moins la tête de ladite broche. De manière préférentielle, la base d'appui est en copolymère d'acide polylactique et de caprolactone tandis que la broche de fixation est en acide polylactique lévogyre et dextrogyre (PLDL)

A titre d'exemple préféré, mais non exhaustif, la base d'appui a été réalisé à partir d'un copolymère 70/30 L-lactide/ ε-caprolactone, ayant un module d'élasticité sous tension de 0,02 à 0,04 GPa, une résistance à la tension de 18 à 22 MPa et un allongement à la rupture supérieure à 100% et la broche de fixation a été réalisée à partir d'un poly(DL-lactide) , ayant un module d'élasticité sous tension de 3,1 à 3,7 GPa, une résistance à la tension de 45 à 55 MPa et un allongement à la rupture de 2 à 6%.

Le diamètre D1 de la base d'appui doit être en adéquation avec les dimensions de l'os à réparer. Il est bien sûr possible que le praticien ait à disposition un jeu complet de bases d'appui de différents diamètres dans lequel il sélectionne sur place, lors de l'intervention, celui qui convient. Cependant, pour éviter d'avoir en stock un nombre important de bases d'appui, la solution proposée par la présente invention est de mettre à la disposition du praticien un instrument de découpe lui permettant de réaliser sur place, à partir d'une base d'appui de grand diamètre D0, la découpe éventuelle de ladite base d'appui lui permettant d'atteindre le diamètre D1 souhaité. Il suffit donc d'avoir en stock un nombre suffisant de base d'appui de ce grand diamètre D0.

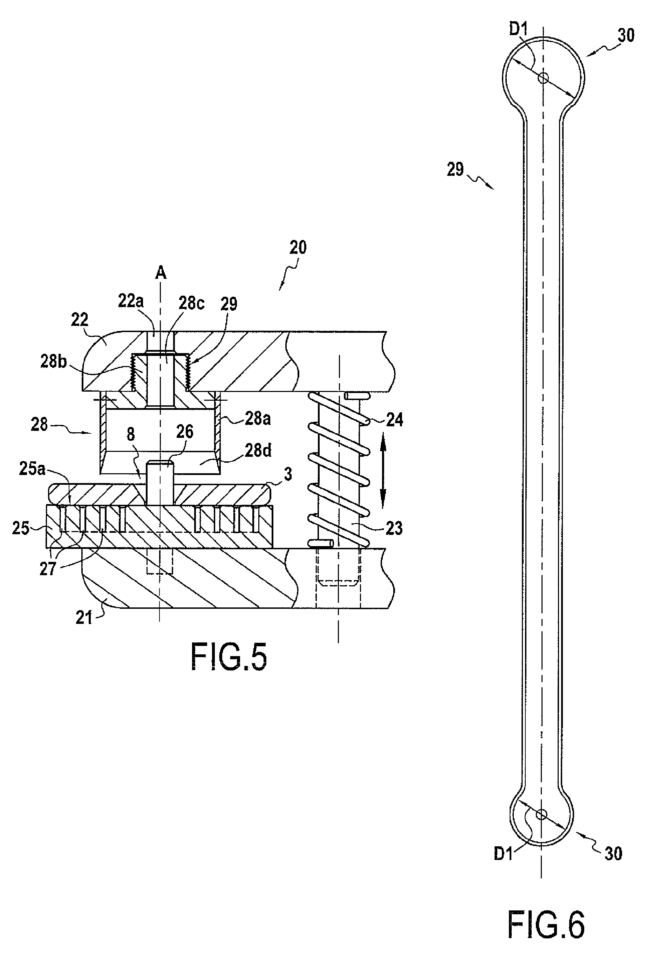

Dans l'exemple illustré à la figure 5, l'instrument de découpe est une pince 20, dont les deux mors 21,22 sont à déplacement parallèle, étant solidarisés l'un à l'autre par une colonne 23 transversale qui est entourée d'un ressort de rappel 24. Lors de l'actionnement de la pince 20, l'un des deux mors coulisse le long de la colonne de sorte que les deux mors 21,22 ont constamment une disposition parallèle.

Le mors inférieur 21 est muni d'une platine 25 circulaire surmontée selon son axe A de symétrie d'un ergot 26 de centrage pour le positionnement précis de la base d'appui 3, le diamètre le plus petit de l'ouverture centrale 8 étant égal ou très légèrement supérieur à celui de l'ergot 26. La face supérieure 25a de la platine est pourvue d'un jeu de rainures 27 circulaires et concentriques de même axe A, de différents diamètres. On a représenté sur la figure 5 quatre, mais de préférence la platine comportera six rainures, de diamètre moyen de 8,10,12,14,16 et 18 mm.

La pince 20 est équipée d'autant d'outils de coupe 28 que de rainures 27, chaque outil de coupe 28 correspondant à un diamètre D1 donné et étant adaptable sur le mors supérieur 22. Cet outil 28 comporte une lèvre 28a circulaire coupante et un épaulement axial arrière 28 b, qui est fileté et apte à coopérer avec le taraudage d'un trou 29 formé dans le mors supérieur 22 selon l'axe A. Lorsque l'outil 28 est mis en place par vissage sur le mors supérieur 22, la lèvre 28a est juste à l'aplomb de la rainure 27 formée dans la platine 25 qui correspond au diamètre D1 de l'outil 28.

Après avoir choisi la valeur du diamètre D1 à l'aide du gabarit 29, illustré à la figure 8, le praticien prend l'outil de coupe 28 du diamètre D1 correspondant, le fixe sur le mors supérieur 22, place sur la platine une base d'appui 3 de grand diamètre D0 sur la face supérieure 21a du mors inférieur 21 et actionne la pince 20, ce qui entraîne le déplacement parallèle des deux mors 21,22 et la pénétration de la lèvre 28a à travers la base d'appui 3 et dans la rainure 27. Après écartement des deux mors sous l'effet du ressort 24, la base d'appui découpée au diamètre D1 reste logée à l'intérieur de la lèvre 28a. Pour faciliter son extraction, de préférence, il est prévu un premier trou axial débouchant 28c dans l'outil de coupe 28, y compris dans l'épaulement 28b arrière fileté, et un second trou axial débouchant 22a dans le mors supérieur 22. L'ensemble chirurgical de l'invention comporte également d'un chasse-rondelle, munie d'une tige apte à passer dans les deux trous axiaux 22a et 28c pour repousser la base d'appui découpée hors de l'espace intérieur 28d de l'outil 28.

Le gabarit 29 est une simple plaque allongée, sans aucun angle vif, terminée, à ses deux extrémités, par deux portions circulaires 30 de diamètres D1 différents. La valeur du diamètre fait l'objet d'un marquage laser à proximité immédiate de la portion 30 correspondante, respectivement 8 et 10 mm dans l'exemple illustré. Chaque portion circulaire 30 est percée d'un trou axial pour le placement adapté du gabarit sur l'os, sur lequel aura été préalablement fixée une tige de positionnement.

La découpe au diamètre D1 requis de la base d'appui 3 grâce à un tel instrument de coupe 20 manuel ne peut s'envisager que parce que le matériau constitutif de la base d'appui 3 présente la déformabilité dont il a été question ci-dessus. De plus le déplacement parallèle des deux mors de la pince est nécessaire si l'on veut une coupe franche de la base d'appui.

## Revendications

1. Dispositif implantable pour la réfection d'une articulation phalangienne ou métacarpo-phalangienne présentant une déficience en cartilage, ledit dispositif comportant une broche de fixation, pour sa fixation dans l'un des os de l'articulation, surmontée d'une base d'appui apte à remplacer le cartilage déficient,
**caractérisé en ce que**:
- la broche de fixation (2) étant formée d'une tête (4) et d'une tige (5), au moins la tête (4) de la broche et la base d'appui (3) sont dans des matériaux différents, celui de la base d'appui (3) étant plus déformable sous contrainte que celui de la tête (4) ;
- la base d'appui (3) présente une ouverture centrale (8) pour l'insertion, par emboîtement à force, et le blocage de la tête de la broche de fixation (4) ;
- les configurations de l'ouverture centrale (8) de la base d'appui (3) et de la tête (4) de la broche (2) de fixation sont déterminées en sorte que la base d'appui (3), lors de son emboîtement à force sur la tête, se déforme pour adopter une courbure concave de rayon R1.

2. Dispositif selon la revendication 1 **caractérisé en ce que** la tête (4) de la broche (2) de fixation a sa face supérieure (4a) qui présente une courbure concave de rayon R2, sensiblement égal à R1.

3. Dispositif selon l'une des revendications 1 ou 2 **caractérisé en ce que** la base d'appui (3) et la broche de fixation sont toutes deux dans des matériaux résorbables, notamment la base d'appui en un copolymère d'acide polylactique et de caprolactone et la tige de fixation en acide polylactique de préférence la base d'appui étant en 70/30 L-lactide/ε-caprolactone et la broche de fixation en poly(DL-lactide).

4. Dispositif selon l'une des revendications 1 à 3 **caractérisé en ce que** la tête (4) de la broche (2) de fixation est reliée à la tige de fixation (5) par un épaulement annulaire (9b), formant butée pour la face inférieure (3b) de la base d'appui (3), lors de l'assemblage, et **en ce que** ladite tête (4) a, en section transversale, des dimensions externes qui sont, dans la zone proche dudit épaulement (9b), supérieures à celles internes de l'ouverture centrale (8) et qui sont, dans la zone opposée audit épaulement (9b), inférieures à celles internes de l'ouverture centrale (8).

5. Dispositif selon la revendication 4 **caractérisé en ce que** la tête de la broche de fixation comporte, à proximité immédiate de l'épaulement annulaire (9), une première portion cylindrique (10) de hauteur H1, prolongée par une seconde portion tronconique (11) divergente de hauteur H2 et d'angle au sommet A2 et l'ouverture centrale (8) de la base d'appui comporte une première portion cylindrique (12) de hauteur H'1 supérieure à H1 et une seconde portion tronconique (13) de hauteur H'2 et d'angle au sommet A'2 supérieur à A'1.

6. Dispositif selon la revendication 5 **caractérisé en ce que** A2 est de l'ordre de 60° et A'2 de l'ordre de 90°

7. Dispositif selon l'une des revendications 1 à 6 **caractérisé en ce que** la base d'appui (3) est une simple rondelle annulaire, dont les deux faces supérieure (3a) et inférieure (3b) sont parallèles, avant l'assemblage avec la broche (2) de fixation, le bord périphérique (3c) de ladite rondelle étant de préférence arrondi.

8. Dispositif selon l'une des revendications 1 à 7 **caractérisé en ce que** la tige (5) de la broche (2) de fixation comporte des crans (9) pour l'ancrage dans l'os et **en ce que** le cran (9a) le plus proche de la tête de la tige fait office de butée pour la face inférieure (3b) de la base d'appui (3) lors de l'assemblage des deux pièces.

9. Ensemble chirurgical comportant d'une part un dispositif selon la revendication 7 dans lequel la base d'appui est une rondelle annulaire de diamètre D0 et d'autre part un instrument de découpe apte à réaliser la découpe de ladite rondelle selon un diamètre D1 inférieur à D0.

10. Ensemble selon la revendication 9 **caractérisé en ce qu'**il comporte un jeu de gabarits (29) destiné à permettre au praticien de mesurer le diamètre D1.

11. Ensemble selon l'une des revendications 9 et 10 **caractérisé en ce que** l'instrument de découpe est une pince emporte-pièce (20) dont un premier mors (21) est muni d'une platine circulaire (25) équipée d'un ergot (26) de centrage pour le placement de la base d'appui (3) et dont le second mors (22) est muni d'un outil de coupe (28) à lèvre coupante circulaire (28a) et **en ce que** les deux mors (21,22) ont un déplacement parallèle.
